# EUROPEAN PATENT SPECIFICATION

(11) **EP 1 014 088 B1**
(45) Date of publication and mention of the grant of the patent: **08.03.2006**
(21) Application number: 99124304.9
(22) Date of filing: 06.12.1999
(51) Int. Cl.: G01N 33/49

(54) **Device and method for separating components of a fluid sample**
Vorrichtung und Verfahren zur Trennung von Bestanteilen einer flüssigen Probe
Appareil et procédé pour séparer des constituants d'un échantillon liquide

(30) Priority: 05.12.1998 US 110937 P
(43) Date of publication of application: 28.06.2000
(73) Proprietor: Becton Dickinson and Company, Franklin Lakes, New Jersey 07417 (US)
(72) Inventor: DiCesare, Paul C., Norwalk, Connecticut 06851 (US); Cohen, Richmond, Wanaque, New Yersey 07465 (US)
(74) Representative: von Kreisler, Alek

(56) References cited:
- EP-A- 0 638 804
- US-A- 4 152 270
- US-A- 4 877 520

## Description

### BACKGROUND OF THE INVENTION

### 1. Field of the Invention

This invention relates to a device and method for separating heavier and lighter fractions of a fluid sample. More particularly, this invention relates to a .device and method for collecting and transporting fluid samples whereby the device and fluid sample are subjected to centrifugation to cause separation of the heavier fraction from the lighter fraction of the fluid sample.

### 2. Description of Related Art

Diagnostic tests may require separation of a patient's whole blood sample into components, such as serum or plasma, the lighter phase component, and red blood cells, the heavier phase component. Samples of whole blood are typically collected by venipuncture through a cannula or needle attached to a syringe or an evacuated collection tube. Separation of the blood into serum or plasma and red blood cells is then accomplished by rotation of the syringe or tube in a centrifuge. Such arrangements use a barrier for moving into an area adjacent the two phases of the sample being separated to maintain the components separated for subsequent examination of the individual components.

A variety of devices have been used in collection devices to divide the area between the heavier and lighter phases of a fluid sample.

The most widely used device includes thixotropic gel materials such as polyester gels in a tube. The present polyester gel serum separation tubes require special manufacturing equipment to prepare the gel and to fill the tubes. Moreover, the shelf-life of the product is limited in that over time globules may be released from the gel mass. These globules have a specific gravity that is less than the separated serum and may float in the serum and may clog the measuring instruments, such as the instrument probes used during the clinical examination of the sample collected in the tube. Such clogging can lead to considerable downtime for the instrument to remove the clog.

No commercially available gel is completely chemically inert to all analytes. If certain drugs are present in the blood sample when it is taken, there can be an adverse chemical interaction with the gel interface.

Therefore, a need exists for a separator device that (i) is easily used to separate a blood sample; (ii) is independent of temperature during storage and shipping; (iii) is stable to radiation sterilization; (iv) employs the benefits of a thixotropic gel barrier yet avoids the many disadvantages of placing a gel in contact with the separated blood components; (v) minimizes cross contamination of the heavier and lighter phases of the sample during centrifugation; (vi) minimizes adhesion of the lower and higher density materials against the separator device; (vii) can be used with standard sampling equipment; (viii) is able to move into position to form a barrier in less time than conventional methods and devices; and (ix) is able to provide a clearer specimen with less cell contamination than conventional methods and devices.

US-A-4,152,270 describes an assembly for separating a fluid sample into a higher specific gravity phase and a lower specific gravity phase, generally corresponding to the first part of claim 1. The assembly comprises a separator element movable within a tube. The separator element constitutes a partitioning device having a specific gravity intermediate those of the light and heavy phases of the blood. The separator element has a resilient sealing member which is of conical or "umbrella" configuration, and a plastic element of different specific gravity connected to the sealing member and having stabilizing members for maintaining the sealing member properly oriented in the tube. The separator element has no hollow core and the elastomeric seal diaphragm does not have a fill septum.

### SUMMARY OF THE INVENTION

It is an object of the invention to provide an assembly that fulfils the above-mentioned requirements.

The assembly of the present invention is defined by claim 1.

The invention refers further to a method for separating a fluid sample into a higher specific gravity phase and a lower specific gravity phase. This method is defined by claim 4.

Desirably, the assembly of the present invention comprises a plurality of constituents. Preferably, the assembly comprises a container and a composite element.

Most preferably, the container is a tube and the composite element is a separator arranged to move in the tube under the action of centrifugal force in order to separate the portions of a fluid sample. Preferably, the separator is a radically deformable seal plug.

Most preferably, the separator is used within a container such as a tube. The tube comprises an open end, a closed end and a sidewall extending between the open end and closed end. The sidewall comprises an outer surface and an inner surface. The tube further comprises a closure disposed to fit in the open end of the tube with a resealable septum. Preferably, the separator element is releaseably positioned at the open end of the tube with the bottom area of the closure.

Most preferably, the separator consists of two components. Preferably, the composite element comprises an overall specific gravity at a target specific gravity of σ₁ The target specific gravity is that required to separate a fluid sample into at least two phases. Preferably, the separator comprises at least two or more regions of differing specific gravities. Preferably, at least one of the regions is higher than the target specific gravity and at least one of the regions is lower than the target specific gravity.

Desirably, the separator comprises a low density plastic body and a high density elastomeric seal diaphragm. The separator as a whole is sized to fit releasably within the tube with an interference fit against the inner surface of the sidewall of the tube.

Preferably, the plastic body has a hollow core or a central passage and a hollow chamber surrounding the hollow core. The plastic body has a specific gravity of about 1.1 to about 7.9.

Desirably, the plastic body is a substantially rigid moldable thermoplastic material such as polystyrene, polyethylene, polypropylene, and mixtures thereof that is inert to the fluid sample of interest.

Desirably, the elastomeric seal diaphragm comprises a fill septum to facilitate needle penetration during the blood fill cycle and an annular skirt that is able to deform during the centrifugation process and reduce the overall diameter of the separator.

Desirably, the plastic body is nested within the elastomeric seal diaphragm, whereby the channel of the plastic body is in direct communication with the fill septum of the elastomeric seal diaphragm.

Desirably, the elastomeric seal diaphragm may be comprised of any natural or synthetic elastomer or mixture thereof, that is inert to the fluid sample of interest. Preferably, the elastomeric seal diaphragm is made from an elastomer having a 50% tensile modulus from about 0.1 MPa to about 1.4 MPa.

Preferably, the separator has a density of about 1.028 to about 1.09 g/cc so that the separator will come to rest after centrifugal force, at substantially at the border between the heavier and lighter phases of the fluid sample under consideration, and most preferably between the heavier and lighter phases of a blood sample. Preferably, the separator will function under load created by an applied acceleration of about 300g to about 3000g.

Preferably, the separator is initially secured to the bottom area of the closure. Desirably, the bottom area of the closure includes an integrally molded gripping means for releasably holding the elastomeric seal diaphragm of the separator with the closure until the assembly is subjected to centrifugation at which time the separator is released from the gripping means of the closure. The separator is further fitted with the tube whereby the annular skirt of the elastomeric seal diaphragm, which provides the largest diameter of the separator in its undeformed state, has an interference fit with the inner surface of the sidewall of the tube.

In use, a fluid sample enters the assembly by needle. The needle penetrates the closure and the elastomeric seal diaphragm of the separator. The sample enters the assembly through the needle and through the channel of the plastic body and into the body of the tube. The needle is withdrawn from the assembly and the septum of the closure and the elastomeric seal diaphragm reseal.

The assembly is then subjected to centrifugation. Under centrifugation, the separator is released from the closure. The separator migrates axially down the tube towards the closed end. The migration is facilitated by the density of the plastic body versus the density of the elastomeric seal diaphragm of the separator. The annular skirt of the elastomeric seal diaphragm temporarily deforms under centrifugation, whereby the diameter is reduced eliminating its interference fit with the inner wall surface of the tube. Therefore, a path is developed between the inner wall of the tube and the separator that permits the flow of the low density component past the separator as it migrates down the tube. Migration of the separator terminates when it reaches the position between the lower density liquid component and higher density cellular/solid components, equal to its overall density. Upon terminating centrifugation, the annular skirt of the elastomeric seal diaphragm returns to its undeformed shape, sealing against the inner wall of the tube, thereby creating a barrier between the higher and lower density components of the fluid.

The separator's position at the top of the tube in conjunction with the closure and the elastomeric seal diaphragm's penetrable area into the passage throughway of the separator, provides easy direct loading of the fluid sample into the tube without any obstructions. Thus, the fluid sample is easily delivered into the tube without exposing the uncentrifuged fluid sample to the outer surface area of the separator.

However, it is within the purview of the invention that the separator may be placed at the bottom of the tube as well.

Thus, when the container is subjected to centrifugation, the separator moves away from the grip of the closure and towards a position between the lower and higher specific gravity phases of the fluid sample.

When the fluid sample is blood, the higher specific gravity portion that contains the cellular components is between the separator and the bottom of the container after centrifugation. The lower specific gravity portion that contains the cell free serum fraction or plasma is between the top surface of the separator and the top of the container after centrifugation.

Therefore, at the final position of the separator after centrifugation, the separator is able to substantially eliminate the presence of red blood cells in the lower specific gravity portion and the lower specific gravity portion is substantially free of cellular contamination.

The assembly of the present invention is advantageous over existing separation products that use gel. In particular the assembly of the present invention will not interfere with analytes as compared to gels that may interfere with certain analytes. Another attribute of the present invention is that the assembly of the present invention will not interfere with therapeutic drug monitoring analytes.

Most notably, the time to separate a fluid sample into separate densities is achieved in substantially less time with the assembly of the present invention as compared to assemblies that use gel.

Another notable advantage of the present invention is that fluid specimens are not subjected to low density gel residuals that are at times available in products that use gel.

A further attribute of the present invention is that there is no interference with instrument probes.

Another attribute of the present invention is that samples for blood banking tests are more acceptable than when a gel separator is used.

Another attribute of the present invention is that only the substantially cell-free serum fraction of a blood sample is exposed to the top surface of the separator, thus providing practitioners with a clean sample.

Additionally, the assembly of the present invention does not require any additional steps or treatment by a medical practitioner, whereby a blood or fluid sample is drawn in the standard fashion, using standard sampling equipment.

### DESCRIPTION OF THE DRAWINGS

FIG. 1 is a perspective view of the assembly of the present invention.
FIG. 2 is a longitudinal sectional view of the assembly of FIG. I taken along line 2-2 thereof.
FIG. 3 is a longitudinal sectional view of the assembly of FIG. 1 taken along line 2-2 thereof illustrating fluid delivery into the assembly by a needle.
FIG. 4 illustrates the assembly under centrifugation and the release of the separator from the gripping means of the closure.
FIG. 5 illustrates the assembly after certrifugation and the separation of the liquid sample into higher and lower specific gravities.
FIG. 6 is a perspective view of the separator of the present invention.
FIG. 7 is a longitudinal sectional view of the separator of FIG. 6 taken along line 7-7 thereof.

### DETAILED DESCRIPTION

The present invention may be embodied in other specific forms and is not limited to any specific embodiments described in detail, which are merely exemplary. Various other modifications will be apparent to and readily made by those skilled in the art without departing from the scope and spirit of the invention. The scope of the invention will be measured by the appended claims and their equivalents.

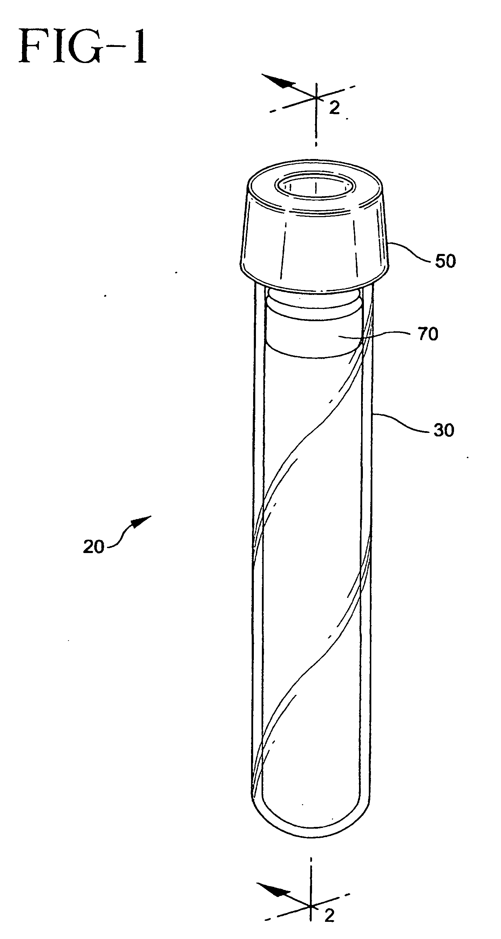

The preferred apparatus of the present invention is illustrated in FIGS. 1 to 5, wherein assembly **20** comprises a tube **30,** a closure **50** and a separator **70.**

Tube **30** has an open end **32** that includes a top edge **33,** a closed end **34** and a sidewall **36** extending between the open end and the closed end. Sidewall **36** has an outer surface **38** and an inner surface **40.** Tube **30** defines a receptacle with a central axis **"A".**

Tube **30** is preferably made from a substantially transparent and rigid material. Suitable materials for the tube include glass, polystyrene, polyethyleneterephthalate, polycarbonate and the like.

Closure **50** is disposed to fit over open end **32** of tube **30.** Closure **50** comprises an annular upper portion **52** which extends over top edge **33** of sidewall 36 and a lower annular portion or skirt **54** of lesser diameter than the annular upper portion **52** which extends into and forms an interference fit with inner surface **40** of sidewall **36** for maintaining stopper **50** in place in open end **32.**

Annular upper portion **52** includes a top surface area **56,** sidewall **58** that converges from surface area **56** towards upper well area **60.** Well area **60** is most preferably a thin diaphragm or a self sealing septum for directing and receiving the point of a needle to be inserted into and through the stopper. The self sealing septum material allows penetration by a piercing element such as a needle and then reseals when the piercing element is withdrawn.

An annular ledge or abutment **57** separates annular upper portion **52** and lower annular portion **54.** Located on bottom surface **68** of lower annular portion **54** are gripping means **69** that are used to initially align and hold the separator in the assembly.

Preferably, the closure may be made of natural rubber elastomer, synthetic thermoplastic and thermoset elastomeric materials. Preferably, the closure is made of a resilient elastomeric material whereby the septum is self-sealing.

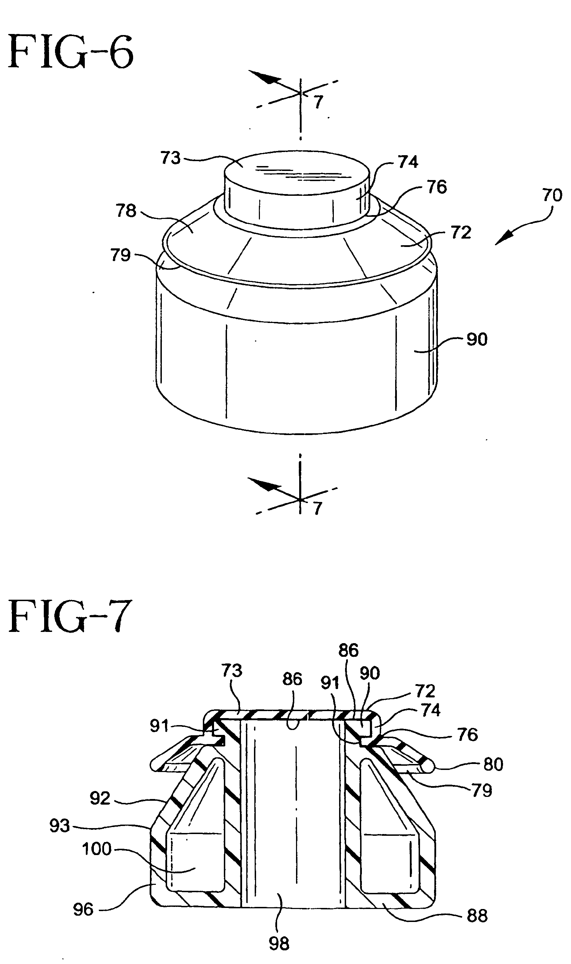

As shown in FIGS. 6 and 7, separator **70** is a dual separator assembly including a molded seal body **90** and an elastomeric hat shaped flexible seal diaphragm **72.** Flexible seal diaphragm **72** includes a top surface **73,** an annular skirt **74** extending downwardly from the top surface to a junction **76** and a flanged annular skirt **78** extending outwardly and downwardly from the junction to a stop surface **79** with a rounded end **80.** Top surface **73** is most preferably a self sealing septum.

Seal body **90** includes a top surface **86,** a bottom surface **88,** an annular upper portion **90** extending from the top surface to an indented junction **91,** a flanged outer sidewall **92** extending downwardly and outwardly and tapers towards a junction **93,** and a second outer sidewall **96** extending from junction **93** downwardly to bottom surface **88.** Inside of seal body **84** is a passageway **98** and a cavity **100.** Passageway **98** extends from top surface **86** to bottom surface **88.** Passageway **98** mates with top surface **73** of annular skirt **74** of flexible seal diaphragm **72** whereby when a piercing element goes through top surface **73** it will extend directly into passageway **98** without any interference.

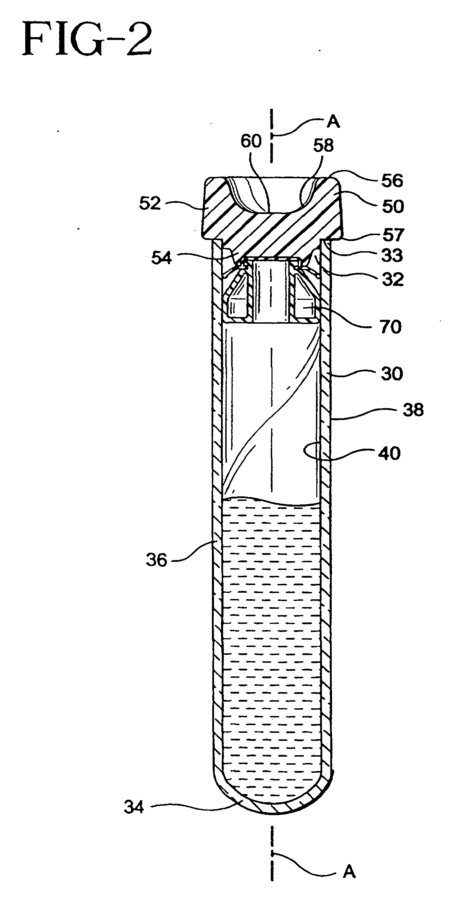

As shown in FIG. 7, annular upper portion **90** of seal body **84** is nested in annular skirt **74** of flexible seal diaphragm **72.** Friction fit, an adhesive or the like may be used to maintain the nesting of the two components. The separator is then held at the top end of the tube by gripping means **69** of the closure. As shown in FIG. 2, rounded end **80** of flexible seal diaphragm **72** and the inner wall of the tube form an interference fit. The seal body does not interfere with the inner wall of the tube as shown in FIG. 2, because the diameter of flanged annular skirt **78** of flexible seal diaphragm **72** is larger than the diameter of seal body **90.**

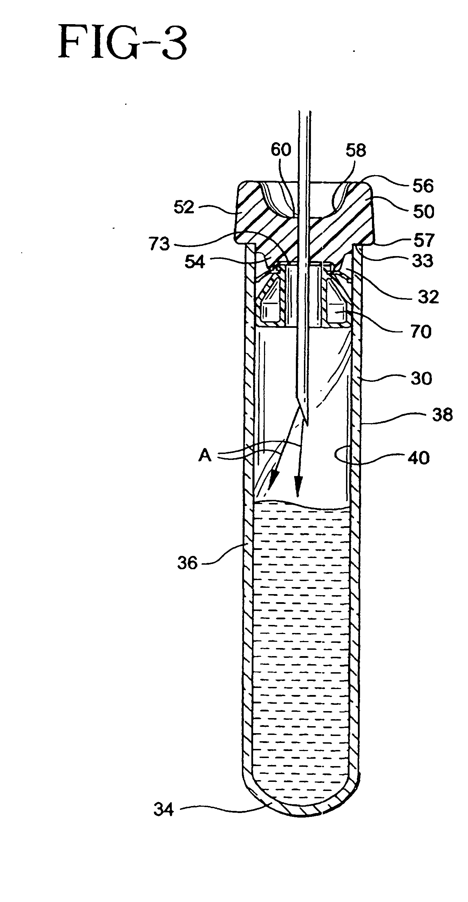

As shown in FIG. 3, a fluid sample A is delivered to the tube by a needle that penetrates closure **50** in upper well area **60** and then top surface **73** of flexible seal diaphragm **72.** For purposes of illustration only, the fluid sample is blood. The fluid sample is delivered into the axial passageway of the separator so that the fluid sample is introduced between closed end **34** of the tube and the separator whereby the outer surface of all components of the separator are substantially free of any contact with the fluid sample.

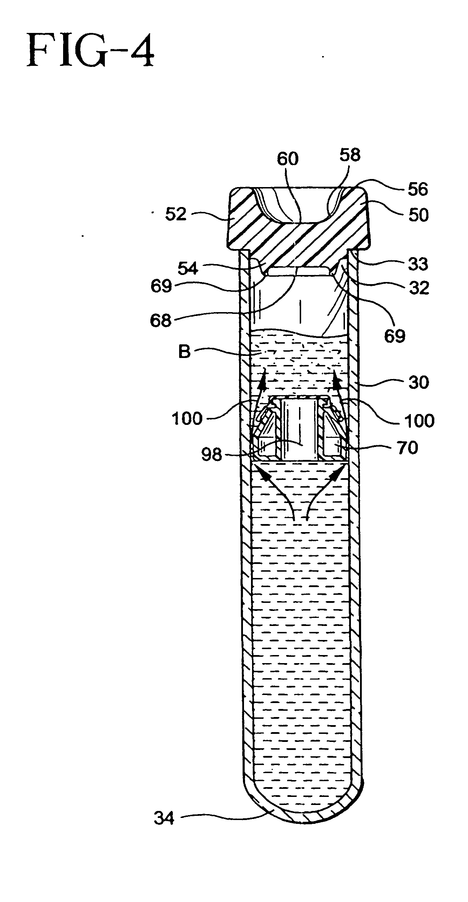

As shown in FIG. 4 when assembly **20** is subjected to centrifugation or axial centrifugation force, separator **70** releases from gripping means **69** of closure **50** and descends towards closed end **34** of tube **30.** As the separator descends, a lower specific gravity fraction **B** of fluid sample **A** moves upwardly past the separator.

As shown in FIG. 4 as the separator descends, flanged annular skirt **78** of the elastomeric seal deforms, reducing its diameter and eliminating its interference with the inner wall of the tube. This opens up a path **100** between the tube and the separator, permitting the flow of the fluid past the separator as the separator migrates down the tube. The residual low density component inside passageway **98** of the separator will migrate downwardly and upwardly past the separator.

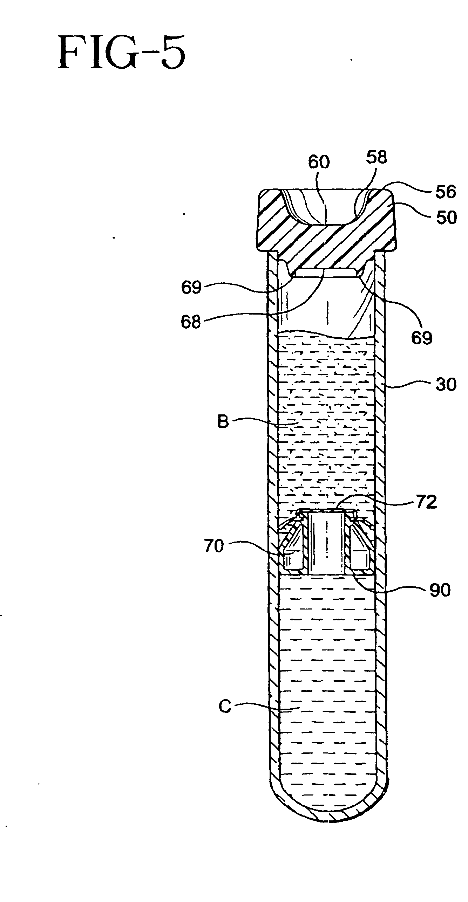

As illustrated in FIG. 5, after centrifugation is complete, centrifugation is terminated, the elastomeric seal diaphragm returns to its undeformed shape, sealing against the inner wall of the tube, whereby separator **70** serves as a divider between lower specific gravity portion **B** and higher specific gravity portion **C** of the fluid sample.

Tube **30** is compatible with most of the numerous additives used in sample collection tubes such as citrates, silicates, EDTA and the like that are used to condition the sample either to facilitate or retard clotting, or to preserve the sample for a particular analysis. It is within the purview of this invention that one or more additives may be used in the present invention for particular applications.

## Claims

1. An assembly for separating a fluid sample into a higher specific gravity phase and a lower specific gravity phase comprising:
a separator element (70) comprising a low density plastic body (90) and a high density elastomeric seal diaphragm (72);
**characterized in that**
said plastic body (90) comprises a hollow core and a hollow chamber (100) surrounding said hollow core;
said elastomeric seal diaphragm (72) comprises a fill septum (73) and an annular skirt (74); and
said plastic body (90) is nested with said elastomeric seal diaphragm (72) whereby said hollow core (98) of said plastic body is in direct communication with said fill septum of said elastomeric seal diaphragm (72).

2. The assembly of claim 1, further comprising:
a tube (30) comprising an open end (32), a closed end (34), an inner diameter, an outer diameter and a sidewall (36) extending between said open end and said closed end;
said sidewall (36) comprising an outer surface (38) and an inner surface (40);
a closure (50) disposed in said open end of said tube;
said separator element being movable axially in said tube (30) under the action of centrifugal force;
said separator element (70) providing selectively an annular seal and an open passage (100) there around in response to pressure differentials in said tube above and below said separator;
said elastomeric seal diaphragm (72) comprising an annular skirt (78) that provides an interference fit with said inner surface (40) of said sidewall (36) of said tube (30) in response to alternating equal and different pressures above and below said separator.

3. The assembly of Claim 2 wherein said separator (70) is located at said open end of said tube (30) by an interference fit between said elastomeric seal diaphragm (72) and said inner diameter of said tube (30).

4. A method for separating a fluid sample into a higher specific gravity phase and a lower specific gravity phase, comprising the steps of:
(a) providing a tube (30) comprising an open end (32), a closed end (34), an inner diameter, an outer diameter and a sidewall extending between said open end and said closed end and comprising an outer surface (38) and an inner surface (40);
(b) providing a closure (50) disposed in said open end of said tube comprising a resealable septum;
(c) providing a separator element (70) comprising an elastomeric seal diaphragm (72) and a plastic body (90) whereby said elastomeric seal diaphragm (72) comprises a fill septum (73) and an annular skirt (78) that provides for selectively providing an interference fit and an open passage with said inner surface (40) of said sidewall (36) of said tube (30) in response to alternating equal and different pressures above and below said separator, and a hollow core extending through said plastic body; said plastic body (90) is nested with said elastomeric seal diaphragm (72) whereby said hollow core of said plastic body is in direct communication with said fill septum of said elastomeric seal diaphragm;
(d) providing said separator element (70) in said tube (30) whereby said elastomeric seal diaphragm (72) comprises an interference fit with said inner wall (40);
(e) providing a needle that penetrates said closure (50) and said septum (73) of said elastomeric seal diaphragm (72);
(f) delivering a fluid sample to said tube (30) whereby said sample enters through said needle and through said septum (73) and said hollow core of said plastic body and then into said body of said tube (30);
(g) removing said needle from said assembly whereby said septum of said closure (50) and said elastomeric seal diaphragm (72) reseals;
(h) subjecting said tube (30) with said separator element (70) to centrifugation whereby said elastomeric seal diaphragm (72) separates from said inner wall of said tube and said separator migrates axially in said tube whereby the low density component (B) of the sample migrates down the tube; and -
(i) terminating said centrifugation whereby said elastomeric seal diaphragm (72) expands to its undeformed shaping, sealing against said inner wall (40) of said tube (30), thereby creating a barrier between said higher and lower density components of said fluid sample.

## Patentansprüche

1. Anordnung zum Separieren einer Flüssigkeitsprobe in eine Phase höherer Dichte und eine Phase geringerer Dichte, mit:
einem Separatorelement (70) mit einem Kunststoffkörper (90) geringer Dichte und einer elastomeren Dichtungsmembran (72) hoher Dichte;
**dadurch gekennzeichnet, daß**
der Kunststoffkörper (90) einen hohlen Kern und eine den hohlen Kern umgebende hohle Kammer (100) aufweist;
die elastomere Dichtungsmembran (72) ein Füllseptum (73) und eine Ringschürze (74) aufweist; und
der Kunststoffkörper (90) mit der elastomeren Dichtungsmembran (72) verschachtelt ist, wodurch der hohle Kern (98) des Kunststoffkörpers in direkter Verbindung mit dem Füllseptum der elastomeren Dichtungsmembran (72) steht.

2. Anordnung nach Anspruch 1, ferner mit:
einem Röhrchen (30) mit einem offenen Ende (32), einem geschlossenen Ende (34), einem Innendurchmesser, einem Außendurchmesser und einer sich zwischen dem offenen Ende und dem geschlossenen Ende erstreckenden Flächenwand (36);
wobei die Flächenwand (36) eine Außenfläche (38) und eine Innenfläche (40) aufweist;
einem im offenen Ende des Röhrchens angeordneten Verschluß (50);
wobei das Separatorelement in dem Röhrchen (30) unter Einwirkung von Zentrifugalkraft axial bewegbar ist;
wobei das Separatorelement (70) in Reaktion auf oberhalb und unterhalb des Separators herrschende Druckunterschiede in dem Röhrchen wahlweise eine ringförmige Dichtung und einen offenen Durchlaß (100) um diesen herum bildet;
wobei die elastomere Dichtungsmembran (72) eine ringförmige Schürze (78) aufweist, die in Reaktion auf abwechselnd gleiche und unterschiedliche Drücke oberhalb und unterhalb des Separators eine Preßpassung mit der Innenfläche (40) der Flächenwand (36) des Röhrchens (30) bildet.

3. Anordnung nach Anspruch 2, bei der der Separator (70) an dem offenen Ende des Röhrchens (30) durch eine Preßpassung zwischen der elastomeren Dichtungsmembran (72) und dem Innendurchmesser des Röhrchens (30) angeordnet ist.

4. Verfahren zum Separieren einer Flüssigkeitsprobe in eine Phase mit höherer Dichte und eine Phase mit geringerer Dichte, mit den folgenden Schritten:
(a) Vorsehen eines Röhrchens (30) mit einem offenen Ende (32), einem geschlossenen Ende (34), einem Innendurchmesser, einem Außendurchmesser und einer sich zwischen dem offenen Ende und dem geschlossenen Ende erstreckenden Flächenwand (36), die eine Außenfläche (38) und eine Innenfläche (40) aufweist;
(b) Vorsehen eines im offenen Ende des Röhrchens angeordneten Verschlusses (50) mit einem wieder abdichtbaren Septum;
(c) Vorsehen eines Separatorelements (70) mit einer elastomeren Dichtungsmembran (72) und einem Kunststoffkörper (90), wobei die elastomere Dichtungsmembran (72) ein Füllseptum (73) und eine Ringschürze (78), die in Reaktion auf abwechselnd gleiche und unterschiedliche Drücke oberhalb und unterhalb des Separators wahlweise eine Preßpassung oder einen offenen Durchlaß mit der Innenfläche (40) der Flächenwand (36) des Röhrchens (30) bildet, und einen sich durch den Kunststoffkörper erstreckenden hohlen Kern aufweist; der Kunststoffkörper (90) ist mit der elastomeren Dichtungsmembran (72) verschachtelt, wodurch der hohle Kern des Kunststoffkörpers in direkter Verbindung mit dem Füllseptum der elastomeren Dichtungsmembran steht;
(d) Anordnen des Separatorelements (70) in ein Röhrchen (30),wobei die elastomere Dichtungsmembran (72) eine Preßpassung mit der Innenwand (40) bildet;
(e) Vorsehen einer Nadel, welche den Verschluß (50) und das Septum (73) der elastomeren Dichtungsmembran (72) durchdringt;
(f) Zuführen einer Flüssigkeitsprobe in das Röhrchen (30), wobei die Probe durch die Nadel, das Septum (73) und den hohlen Kern des Kunststoffkörpers und anschließend in den Körper des Röhrchens (30) eintritt;
(g) Entfernen der Nadel aus der Anordnung, wobei das Septum des Verschlusses (50) und die elastomere Dichtungsmembran (72) sich wieder abdichten;
(h) Zentrifugieren des Röhrchens (30) mit dem Separatorelement (70), wobei sich die elastomere Dichtungsmembran (72) von der Innenwand des Röhrchens löst und der Separator axial in dem Röhrchen wandert, wobei der Probenbestandteil (B) mit geringer Dichte in dem Röhrchen nach unten wandert; und
(i) Beenden des Zentrifugierens, wobei sich die elastomere Dichtungsmembran zu ihrer unverformten Form ausdehnt und die Innenwand (40) des Röhrchens (30) abdichtet, wodurch eine Sperre zwischen den eine hohe bzw. eine geringe Dichte aufweisenden Bestandteilen der Flüssigkeitsprobe gebildet wird.

## Revendications

1. Ensemble pour la séparation d'un échantillon de fluide en une phase de plus grande densité et une phase de plus faible densité, comprenant :
- un élément de séparateur (70) comprenant un corps en matière plastique de faible densité (90) et un diaphragme d'étanchéité en élastomère de haute densité (72) ;
**caractérisé en ce que** :
- ledit corps en matière plastique (90) comprend un noyau creux et une chambre creuse (100) entourant ledit noyau creux ;
- ledit diaphragme d'étanchéité en élastomère (72) comprend un septum de remplissage et une jupe annulaire (74) ; et
- ledit corps en matière plastique (90) est incorporé avec ledit diaphragme d'étanchéité en élastomère (72), ledit noyau creux (98) dudit corps en matière plastique étant ainsi en communication directe avec ledit septum de remplissage dudit diaphragme d'étanchéité en élastomère (72).

2. Ensemble selon la revendication 1, comprenant, de plus :
- un tube (30) comprenant une extrémité ouverte (32), une extrémité fermée (34), un diamètre intérieur, un diamètre extérieur et une paroi latérale (36) s'étendant entre ladite extrémité ouverte et ladite extrémité fermée ;
- ladite paroi latérale (36) comprenant une surface externe (38) et une surface interne (40) ;
- une pièce de fermeture (50) disposée dans ladite extrémité ouverte dudit tube ;
ledit élément de séparateur étant mobile, de façon axiale, dans ledit tube (30) sous l'action de la force centrifuge ;
ledit élément de séparateur (70) constituant, de façon sélective, un joint annulaire d'étanchéité et un passage ouvert (100) autour de celui-ci en réponse à des différences de pression dans ledit tube au-dessus et en dessous dudit séparateur ;
ledit diaphragme d'étanchéité en élastomère (72) comprend une jupe annulaire (78) qui présente un ajustement avec serrage avec ladite surface interne (40) de ladite paroi latérale (36) dudit tube (30) en réponse à des pressions égales et différentes, de façon alternée, au-dessus et en dessous dudit séparateur.

3. Ensemble selon la revendication 2, dans lequel ledit séparateur (70) est positionné sur ladite extrémité ouverte dudit tube (30) par ajustement avec serrage entre ledit diaphragme d'étanchéité en élastomère (72) et ledit diamètre intérieur dudit tube (30).

4. Procédé de séparation d'un échantillon de fluide en une phase de plus grande densité et une phase de plus faible densité, comprenant les étapes suivantes :
a) la fourniture d'un tube (30) comprenant une extrémité ouverte (32), une extrémité fermée (34), un diamètre intérieur, un diamètre extérieur et une paroi latérale s'étendant entre ladite extrémité ouverte et ladite extrémité fermée et comprenant une surface externe (38) et une surface interne (40) ;
b) la fourniture d'une pièce de fermeture (50) disposée dans ladite extrémité ouverte dudit tube comprenant un septum amovible ;
c) la fourniture d'un élément de séparateur (70) comprenant un diaphragme d'étanchéité en élastomère (72) et un corps en matière plastique (90), ledit diaphragme d'étanchéité en élastomère (72) comprenant ainsi un septum de remplissage (73) et une jupe annulaire (78) qui est prévue pour constituer, de façon sélective, un ajustement avec serrage et un passage ouvert avec ladite surface interne (40) de ladite paroi latérale (36) dudit tube (30) en réponse à des pressions égales et différentes, de façon alternée, au-dessus et en dessous dudit séparateur, et un noyau creux traversant ledit corps en matière plastique ; ledit corps en matière plastique (90) est incorporé avec ledit diaphragme d'étanchéité en élastomère (72), ledit noyau creux dudit corps en matière plastique étant ainsi en communication directe avec ledit septum de remplissage dudit diaphragme d'étanchéité en élastomère ;
d) la fourniture dudit élément de séparateur (70) dans ledit tube (30), ledit diaphragme d'étanchéité en élastomère (72) comprenant un ajustement avec serrage avec ladite paroi interne (40) ;
e) la fourniture d'une aiguille pénétrant ladite pièce de fermeture (50) et ledit septum (73) dudit diaphragme d'étanchéité en élastomère (72) ;
f) la délivrance d'un échantillon de fluide audit tube (30), ledit échantillon pénétrant ainsi à travers ladite aiguille et ledit septum (73), ledit noyau creux dudit corps en matière plastique puis dans ledit corps dudit tube (30) ;
g) le retrait de ladite aiguille dudit ensemble, ledit septum de ladite pièce de fermeture (50) et ledit diaphragme d'étanchéité en élastomère (72) se refermant ainsi ;
h) la soumission dudit tube (30) avec ledit élément de séparateur (70) à une centrifugation, ledit diaphragme d'étanchéité en élastomère (72) se séparant ainsi de ladite paroi interne dudit tube et ledit séparateur migrant ainsi, de façon axiale, dans ledit tube, le composant de faible densité (B) de l'échantillon descendant ainsi dans le bas du tube ; et
i) l'arrêt de ladite centrifugation, ledit diaphragme d'étanchéité en élastomère (72) se dilatant ainsi vers sa forme non déformée, s'appuyant, de façon étanche, contre ladite paroi interne (40) dudit tube (30), créant ainsi une barrière entre lesdits composants de plus grande densité et de plus faible densité dudit échantillon de fluide.
